# EUROPEAN PATENT APPLICATION

(11) **EP 3 907 290 A1**
(43) Date of publication of application: **10.11.2021**
(21) Application number: 19889198.8
(22) Date of filing: 29.11.2019
(51) Int. Cl.: C12N 15/81, C12P 23/00, C12N 9/04, C12N 9/02, C12N 9/10

(54) **MICROORGANISM PRODUCING BIORETINOL AND METHOD FOR PRODUCING BIORETINOL USING SAME**

(30) Priority: 30.11.2018 KR 20180152617
(71) Applicant: Biosplash Co., Ltd., Gyeonggi-do 16006 (KR)
(72) Inventor: YU, Byung Jo, Suwon-si, Gyeonggi-do 16582 (KR); JANG, In Seung, Busan 48046 (KR); JANG, Ji Yeon, Seoul 06651 (KR)
(74) Representative: Icosa
(86) International application number: PCT/KR2019/016783
(87) International publication number: WO 2020/111890

(57) **Abstract**

Provided are a microorganism for producing retinol, in which retinol biosynthetic genes are introduced; and a method of producing retinol, the method including a step of culturing the microorganism.

The microorganism of the present invention may have an improved ability to produce retinol, and thus it may be efficiently used in producing retinol. Based on the method of producing retinol, the method including the step of culturing the microorganism, the retinol production efficiency may be improved.

## Description

### BACKGROUND OF THE INVENTION

### 1. Field of the Invention

The present invention relates to a microorganism for producing bioretinol, in which beta- carotene and retinol biosynthetic genes are introduced; and a method of producing bioretinol, the method including a step of culturing the microorganism.

### 2. Description of the Related Art

Retinol is a high-value-added raw material that has anti-wrinkle and antioxidant effects. Specifically, retinol and derivatives thereof, which are ingredients that help improve skin wrinkles, are raw materials that provide excellent efficacy in improving wrinkles such that they make up three of the four ingredients listed as raw materials for functional cosmetics in a notification issued by the Ministry of Food and Drug Safety. Therefore, retinol having the above functions may be used as a cosmetic composition having an anti- wrinkle effect.

However, despite such usefulness of retinol, mass production of retinol is not easy.The existing representative method of producing retinol is to produce the final products, retinol and derivatives thereof, through chemical reactions of pentadiene → retinal → retinol after extracting precursors from natural resources. However, the method through such a chemical process has a production cost of 3,500 to 4,000 US dollars per 1 kg, which causes a problem of increasing the production cost of retinol-containing products. Accordingly, in order to achieve more stable production and high yield, a highly efficient retinol production technology through a bio-process is required.

The present inventors have made many efforts to increase the production of retinol, and as a result, they have developed a microorganism capable of more stably producing retinol with high efficiency, and confirmed that the production amount of retinol was increased by culturing the microorganism under various conditions, thereby completing the present invention. When retinol is produced by a yeast fermentation process of the present invention, safety is high because an eco-friendly and safe yeast strain is used. It is also expected to improve price competitiveness, as compared to the existing production technology, because high-efficiency production is possible due to BMCO expression using a multi-copy plasmid.

### SUMMARY OF THE INVENTION

An object of the present invention is to provide a microorganism for producing retinol, in which geranylgeranyl diphosphate synthase (GGPP synthase, crtE), phytoene synthase (crtYB), desaturase (crtI), beta-carotene 15,15'-monooxygenase (β-carotene 15,15'-monooxygenase, BCMO), and retinol dehydrogenase (ybbO) protein activities are enhanced.

Another object of the present invention is to provide a method of producing retinol, the method including a step of culturing the microorganism in a culture medium.

### BRIEF DESCRIPTION OF THE DRAWINGS

FIG. 1 shows the design of biosynthetic metabolic pathways of retinol;
FIG. 2 shows SDS-PAGE confirming integration of crtE(A), crtI(B), and crtYB(C) genes;
FIG. 3 shows PCR results confirming integration of BCMO-SR or BCMO-blh gene;
FIG. 4 shows colony formation (A) and master colony formation (B) confirming integration of BCMO-SR or BCMO-blh gene;
FIG. 5 shows SDS-PAGE confirming integration of BCMO-SR(A) or BCMO-blh(B) gene;
FIG. 6 shows PCR results confirming integration of yybO gene;
FIG. 7 shows HPLC/UV chromatographic analysis measuring production amounts of retinal (A) and retinol (B) of a transformed strain of the present invention;
FIG. 8 shows an ESI-Mass spectrum graph measuring production amounts of retinol of a wild-type strain (A) and the transformed strain (B) of the present invention;

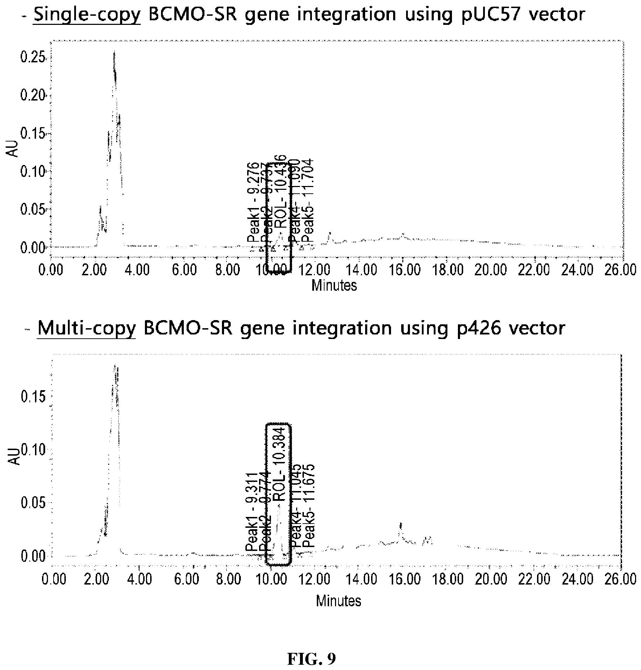

FIG. 9 shows an HPLC graph measuring production amounts of retinol when BMCO genes were introduced using a single-copy plasmid and a multi-copy plasmid; and

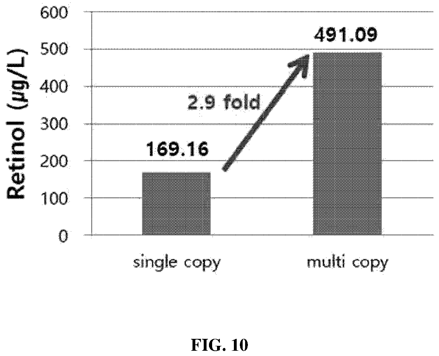

FIG. 10 shows a graph showing a quantitative comparison result of retinol production when the single-copy plasmid or the multi-copy plasmid was used.

### DETAILED DESCRIPTION OF THE PREFERRED EMBODIMENTS

To achieve the above objects, an aspect of the present disclosure provides a microorganism for producing retinol, in which geranylgeranyl diphosphate synthase (GGPP synthase, crtE), phytoene synthase (crtYB), desaturase (crtI), *beta-carotene* 15,15'-monooxygenase (β-carotene 15,15'-monooxygenase, BCMO), and retinol dehydrogenase (ybbO) protein activities are enhanced.

Further, the present invention provides use of the microorganism, in which geranylgeranyl diphosphate synthase (GGPP synthase, crtE), phytoene synthase (crtYB), desaturase (crtI), *beta*-carotene 15,15'-monooxygenase (β-carotene 15,15'- monooxygenase, BCMO), and retinol dehydrogenase (ybbO) protein activities are enhanced, in retinol production.

As used herein, the term "retinol" refers to a type of vitamin A, and retinol has an important role in maintaining original functions of the skin's epidermal cells. Specifically, retinol is the chemical name of vitamin A1, which exists in the intestinal mucosa of animals, and is known to be contained in large amounts in greenish-yellow plants. Retinol is also transformed into retinoic acid as an active form, and allows DNA to express RNA in the cell nuclei present in skin cells to promote cell differentiation, and has efficacy of reducing wrinkles and improving skin elasticity due to its promotion of biosynthesis of collagen, which is a fibrous protein that exists as a fibrous solid between animal cells, and elastin, which is composed of elastic fibers. As described, retinol has been used as a cosmetic ingredient. However, after extracting precursors from natural plant resources, retinol and derivatives thereof, which are final products, are mainly produced through chemical reactions, and such a chemical production method has disadvantages in that purity is low due to high probability of contamination of other impurities, and their production amounts are low to increase the unit cost of production. In contrast, retinol produced using the high-efficiency fermentation production technology of the present invention may be used as a cosmetic composition having effects of preventing skin aging, improving skin elasticity, and improving wrinkles.

As used herein, the term "geranylgeranyl diphosphate synthase (GGPP synthase, crtE)" refers to an enzyme that catalyzes the reversible reaction of the following Reaction Scheme.

### [Reaction Scheme]

farnesyl diphosphate + isopentenyl diphosphate ⇔ geranylgeranyl pyrophosphate As used herein, the term "phytoene synthase (crtYB)", which is one of the enzymes involved in carotenoid biosynthesis, refers to an enzyme that catalyzes the reversible reaction of the following Reaction Scheme, and specifically, it may refer to an enzyme that synthesizes phytoene, but is not limited thereto.

### [Reaction Scheme]

2 geranylgeranyl diphosphate ⇔ 15-*cis*-phytoene + 2 diphosphate As used herein, the term "desaturase (crtI)" refers to an enzyme that catalyzes the reversible reaction of the following Reaction Scheme, and specifically, it may refer to an enzyme that synthesizes lycopene, but is not limited thereto. Further, desaturase may be used interchangeably with phytoene desaturase.

### [Reaction Scheme]

15-*cis*-phytoene + 4 acceptor ⇔ all-*trans*-lycopene + 4 reduced acceptor (Overall Reaction Scheme)
(1a) 15-*cis*-phytoene + acceptor ⇔ all*-trans-*phytofluene + reduced acceptor
(1b) all*-trans-*phytofluene + acceptor ⇔ *all-trans-zeta-carotene* + reduced acceptor
(1c) *all-trans-zeta-carotene* + acceptor ⇔ all-*trans*-neurosporene + reduced acceptor
(1d) all-*trans*-neurosporene + acceptor ⇔ all-*trans*-lycopene + reduced acceptor

As used herein, the term "*beta*-carotene 15,15'-monooxygenase (β-carotene 15,15'-monooxygenase, BCMO)" refers to an enzyme that catalyzes the following Reaction Scheme, and specifically, it may refer to an enzyme that cleaves *beta*-carotene using oxygen molecules to produce two molecules of retinal. *beta*-Carotene 15,15'-monooxygenase may also process *beta*-cryptoxanthin, apocarotenal, 4'*-*apo*-beta-*carotenal, *alpha-carotene,* and gamma-carotene, and it may also be used interchangeably with *beta*-carotene 15,15'-dioxygenase (β-carotene 15,15'-dioxygenase).

### [Reaction Scheme]

### beta-carotene + O₂ → 2 all-trans-retinal

As used herein, the term "retinol dehydrogenase (ybbO)" refers to an enzyme that catalyzes the reversible reaction of the following Reaction Scheme, and *all-trans-* or *cis-*retinol may be a substrate of the enzyme. Two substrates (retinol and NAD⁺) may be used to produce three products (retinal, NADH, and H⁺). It may also be used interchangeably with microsomal retinol dehydrogenase, all-*trans*-retinol dehydrogenase, retinal reductase, and retinene reductase.

### [Reaction Scheme]

### retinol + NAD⁺ ⇔ retinal + NADH + H⁺

The genetic information of geranylgeranyl diphosphate synthase, phytoene synthase, desaturase, *beta*-carotene 15,15'-monooxygenase, and retinol dehydrogenase may be obtained from a public database, for example, GenBank of the National Center for Biotechnology Information (NCBI), *etc.,* but is not limited thereto.

The geranylgeranyl diphosphate synthase, phytoene synthase, and desaturase may be derived from *Xanthophyllomyces dendrorhous,* and the *beta-carotene* 15,15'- monooxygenase and retinol dehydrogenase may be derived from *Halobacterium* sp. NRC-1, marine bacterium 66A03, or *Escherichia* sp. microorganisms, but they are not limited to the origin or sequence thereof, because there may be differences in the amino acid sequence of the active protein depending on the species of a microorganism or the microorganism. In particular, the retinol dehydrogenase may be derived from an *Escherichia* sp. microorganism, specifically, *E. coli,* but is not limited thereto. Specifically, the geranylgeranyl diphosphate synthase may be a protein including an amino acid sequence of SEQ ID NO: 7, the phytoene synthase may be a protein including an amino acid sequence of SEQ ID NO: 8, and the desaturase may be a protein including an amino acid sequence of SEQ ID NO: 9. With regard to the *beta*-carotene 15,15'- monooxygenase, BCMO-SR may be a protein including an amino acid sequence of SEQ ID NO: 10, and BCMO-blh may be a protein including an amino acid sequence of SEQ ID NO: 11. The retinol dehydrogenase may be a protein including an amino acid sequence of SEQ ID NO: 12. However, these are not limited thereto. In the present invention, "protein including an amino acid sequence" may be used interchangeably with "protein consisting of an amino acid sequence".

Further, in the present invention, each of the enzymes may include a protein exhibiting 80% or more, 85% or more, 90% or more, 91% or more, 92% or more, 93% or more, 94% or more, 95% or more, 96% or more, 97% or more, 98% or more, or 99% or more homology to the amino acid sequence, as well as the above-described SEQ ID NO, as long as it has a biological activity identical or corresponding to that of each enzyme. Further, it is obvious that any amino acid sequence with deletion, modification, substitution, or addition in part of the sequence may also be included within the scope of the present disclosure, as long as the amino acid sequence has homology to the above sequence and has a biological activity substantially identical or corresponding to that of the enzyme protein of the above-described SEQ ID NO.

As used herein, the term "homology" refers to a degree of matching with a given amino acid sequence or nucleotide sequence, and the homology may be expressed as a percentage. In the present specification, a homology of a sequence having an activity which is identical or similar to that of a given amino acid sequence or nucleotide sequence is expressed as "% homology". The homology of a sequence may be determined by, for example, a standard software, specifically BLAST 2.0, which calculates the parameters such as score, identity, similarity, *etc.,* or by comparing the sequences in a Southern hybridization experiment under defined stringent conditions, and defining appropriate hybridization conditions is within the skill of the art, and may be determined using a method well known to those skilled in the art *(e.g.,* J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York). The term "stringent conditions" refers to conditions which allow specific hybridization between polynucleotides. For example, such conditions are disclosed in detail in the literature *(e.g.,* J. Sambrook *et al., supra).* Each of the geranylgeranyl diphosphate synthase, phytoene synthase, desaturase, *beta-*carotene 15,15'-monooxygenase, and retinol dehydrogenase of the present invention may include a polynucleotide encoding the amino acid sequence of the above-described SEQ ID NO, or a protein exhibiting 80% or more, 85% or more, 90% or more, 91% or more, 92% or more, 93% or more, 94% or more, 95% or more, 96% or more, 97% or more, 98% or more, or 99% or more homology to the sequence, as long as it has a biological activity identical or corresponding to that of each enzyme. Specifically, a gene encoding the geranylgeranyl diphosphate synthase (GGPP synthase, crtE) may include a nucleotide sequence of SEQ ID NO: 1; a gene encoding the phytoene synthase (crtYB) may include a nucleotide sequence of SEQ ID NO: 2; a gene encoding the desaturase (crtI) may include a nucleotide sequence of SEQ ID NO: 3; with regard to genes encoding the *beta*-carotene 15,15'-monooxygenase (BCMO), a BCMO-SR gene may include a nucleotide sequence of SEQ ID NO: 4, and a BCMO-blh gene may include a nucleotide sequence of SEQ ID NO: 5; and a gene encoding the retinol dehydrogenase may include a nucleotide sequence of SEQ ID NO: 6.

Further, each of the polynucleotides encoding the enzymes may undergo various modifications in the coding region without changing the amino acid sequence of the protein expressed from the coding region, due to codon degeneracy or in consideration of the codons preferred in an organism in which the protein is to be expressed. Therefore, each of the polynucleotides may include any polynucleotide sequence without limitation, as long as it encodes each enzyme protein.

Further, a probe which may be prepared from a known gene sequence, for example, any polynucleotide sequence which hybridizes with a sequence complementary to all or a part of the polynucleotide sequence under stringent conditions to encode the protein having activity of geranylgeranyl diphosphate synthase, phytoene synthase, desaturase, *beta-* carotene 15,15'-monooxygenase, or retinol dehydrogenase enzyme, may be included without limitation.

The "stringent conditions" refer to conditions which allow specific hybridization between polynucleotides. Such conditions are disclosed in detail in the literature *(e.g.,* J. Sambrook *et al., supra).* For example, the stringent conditions may include conditions under which genes having high homology, genes having 40% or more, specifically 90% or more, more specifically 95% or more, much more specifically 97% or more, and particularly specifically 99% or more homology hybridize with each other, while genes having homology lower than the above homology do not hybridize with each other; or may include ordinary washing conditions of Southern hybridization, *i.e.,* washing once, specifically twice or three times, at a salt concentration and a temperature corresponding to 60°C, 1XSSC, 0.1% SDS; specifically 60°C, 0.1XSSC, 0.1% SDS; and more specifically 68°C, 0.1XSSC, 0.1% SSD.

Hybridization requires that two polynucleotides have complementary sequences, although mismatches between bases are possible depending on the stringency of the hybridization. The term "complementary" is used to describe a relationship between nucleotide bases that may hybridize with each other. For example, with respect to DNA, adenosine is complementary to thymine, and cytosine is complementary to guanine. Therefore, the present disclosure may also include an isolated polynucleotide fragment complementary to the entire sequence as well as a polynucleotide sequence substantially similar thereto.

Specifically, a polynucleotide having homology may be detected using hybridization conditions including a hybridization step at a Tₘ value of 55°C under the above-described conditions. Additionally, the Tₘ value may be 60°C, 63°C, or 65°C, but is not limited thereto, and may be appropriately controlled by those skilled in the art depending on the purpose thereof.

The appropriate stringency for hybridizing polynucleotides depends on the length and degree of complementarity of the polynucleotides, and these variables are well known in the art (see Sambrook *et al., supra,* 9.50-9.51, 11.7-11.8).

As used herein, the term "enhancement of activity" means that activity of the enzyme protein is introduced, or the activity is increased, as compared with endogenous activity of a microorganism or activity before modification. The "introduction" of the activity means that activity of a specific protein that is not originally possessed by a microorganism is naturally or artificially exhibited. Specifically, the microorganism, in which activity of the enzyme protein is enhanced, refers to a microorganism, in which activity of the enzyme protein is enhanced, as compared with that of the natural wild-type microorganism or non-transformed microorganism. The enhancement of the activity may include, for example, enhancement by introducing foreign geranylgeranyl diphosphate synthase, phytoene synthase, desaturase, *beta*-carotene 15,15'- monooxygenase, and/or retinol dehydrogenase; or enhancement of the activity of endogenous geranylgeranyl diphosphate synthase, phytoene synthase, desaturase, *beta-* carotene 15,15'-monooxygenase, and/or retinol dehydrogenase, and specifically, the method of enhancing the activity in the present invention may be, for example, performed by way of
1) a method of increasing the copy number of the polynucleotide encoding each enzyme,
2) a method of modifying an expression regulatory sequence to increase expression of each polynucleotide,
3) a method of modifying the polynucleotide sequence on the chromosome to enhance the activity of each enzyme, or
4) a combination of the methods, but the method is not limited thereto.
1) The method of increasing the copy number of the polynucleotide may be performed, but is not particularly limited to, in the form in which the polynucleotide is operably linked to a vector; or by integrating the polynucleotide into the chromosome of a host cell.

In addition, the increase in the copy number may be performed by introducing, into a host cell, a foreign polynucleotide exhibiting the activity of the enzyme or a variant polynucleotide obtained by codon-optimizing the polynucleotide. The foreign polynucleotide may be used without limitation in the origin or sequence thereof, as long as it exhibits activity identical/similar to that of the enzyme. The introducing may be performed using a known transformation method which is appropriately selected by those skilled in the art.When the introduced polynucleotide is expressed in the host cell, the enzyme is produced, and thus its activity may be increased.

Next, 2) the method of modifying an expression regulatory sequence to increase expression of each polynucleotide may be performed, but is not particularly limited to, by inducing a variation in the nucleotide sequence via deletion, insertion, non- conservative or conservative substitution, or any combination thereof so as to further enhance the activity of the expression regulatory sequence, or by replacing the sequence with a nucleotide sequence having a stronger activity. The expression regulatory sequence may include, but is not particularly limited to, a promoter, an operator sequence, a sequence encoding a ribosome-binding site, a sequence for regulating termination of transcription and translation, *etc.*

Specifically, a strong heterologous promoter, instead of the original promoter, may be linked upstream of the polynucleotide expression unit, and examples of the strong promoter may include a CJ7 promoter, a lysCP1 promoter, an EF-Tu promoter, a groEL promoter, an aceA or aceB promoter, *etc.* More specifically, a hxpr2 promoter or a UAS1B promoter is operably linked to improve the expression rate of the polynucleotide encoding each enzyme, but is not limited thereto.

Furthermore, 3) the method of modifying the polynucleotide sequence on the chromosome may be performed, but is not particularly limited to, by inducing a variation in the expression regulatory sequence by deletion, insertion, non-conservative or conservative substitution, or any combination thereof to further enhance the activity of the polynucleotide sequence, or by replacing the sequence with a polynucleotide sequence which is modified to have a stronger activity.

Finally, 4) the method of enhancing the activity by any combination of 1) to 3) may be performed by combining one or more of the method of increasing the copy number of the polynucleotide encoding each enzyme, the method of modifying an expression regulatory sequence to increase expression thereof, and the method of modifying the polynucleotide sequence on the chromosome, and the method of modifying the foreign polynucleotide having the activity of the enzyme or a codon-optimized variant polynucleotide thereof. The polynucleotide may be described as a gene in the case where it refers to an assemblage of polynucleotides capable of carrying out functions. In the present disclosure, polynucleotides and genes may be used interchangeably, and polynucleotide sequences and nucleotide sequences may be used interchangeably.

As used herein, the term "vector" refers to a DNA construct containing a target protein- encoding polynucleotide sequence operably linked to a suitable regulatory sequence so as to be able to express the target protein in a suitable host cell. The regulatory sequence may include a promoter capable of initiating transcription, any operator sequence for regulating the transcription, a sequence encoding a suitable mRNA ribosome binding site, and a sequence for regulating termination of transcription and translation. Once transformed into an appropriate host cell, the vector may replicate or function independently from the host genome, or may integrate into a genome thereof. The vector used in the present disclosure is not particularly limited, as long as it may replicate in a host cell. Any vector known in the art may be used. Examples of the vectors commonly used may include natural or recombinant plasmids, cosmids, viruses, and bacteriophages. For example, pWE15, M13, MBL3, MBL4, IXII, ASHII, APII, t10, t11, Charon4A, Charon21A, *etc.* may be used as a phage vector or cosmid vector. pBR- based, pUC-based, pBluescriptII-based, pGEM-based, pTZ-based, pCL-based, pET-based vectors, *etc.* may be used as a plasmid vector. Specifically, pDZ, pACYC177, pACYC184, pCL, pECCG117, pUC19, pBR322, pMW118, pCC1BAC, pSKH, pRS-413, pRS-414, pRS-415, pBCA, or pYLI vector, *etc.* may be used, but the vector is not limited thereto.

The vector applicable in the present invention is not particularly limited, and a known expression vector may be used. Additionally, a polynucleotide encoding a target protein may be integrated into the chromosome through a vector for chromosomal insertion. The integration of the polynucleotide into the chromosome may be performed using any method known in the art, for example, by homologous recombination, but is not limited thereto. A selection marker for confirming the integration into the chromosome may be further included, or a gene related thereto may be removed. The selection marker is used for selecting a cell transformed with a vector, *i*.*e*., in order to confirm whether or not the target nucleic acid molecules have been inserted, and markers that provide selectable phenotypes, such as drug resistance, auxotrophy, resistance to cytotoxic agents, or expression of surface proteins, may be used. Only the cells expressing the selection markers are able to survive or express other phenotypic traits under the environment treated with selective agents, and therefore, the transformed cells may be selected.

As used herein, the term "transformation" means introduction of a vector including a polynucleotide encoding a target protein into a host cell in such a way that the protein encoded by the polynucleotide is expressed in the host cell. As long as the transformed polynucleotide may be expressed in the host cell, it may be integrated into and located in the chromosome of the host cell or exist extrachromosomally, or it may be in any of the cases. Further, the polynucleotide includes DNA and RNA which encode the target protein. The polynucleotide may be introduced in any form as long as it may be introduced into a host cell and expressed therein. For example, the polynucleotide may be introduced into a host cell in the form of an expression cassette, which is a gene construct including all elements necessary for autonomous expression. The expression cassette may commonly include a promoter operably linked to the polynucleotide, a transcription termination signal, a ribosome binding site, and a translation termination signal. The expression cassette may be in the form of an expression vector capable of self-replication. Further, the polynucleotide may be introduced into a host cell as it is and operably linked to a sequence necessary for its expression in the host cell, but is not limited thereto. The transformation method includes any method of introducing a nucleic acid into a cell, and may be performed by selecting a suitable standard technique known in the art according to the host cell. For example, the transformation may be carried out via electroporation, calcium phosphate (CaPO₄) precipitation, calcium chloride (CaCl₂) precipitation, microinjection, a polyethylene glycol (PEG) technique, a DEAE-dextran technique, a cationic liposome technique, a lithium acetate-DMSO technique, *etc.,* but the method is not limited thereto.

Further, the term "operably linked" means a functional linkage between a promoter sequence which initiates and mediates transcription of the polynucleotide encoding the target protein of the present disclosure and the polynucleotide sequence. The operable linkage may be prepared using a genetic recombinant technique known in the art, and site-specific DNA cleavage and ligation may be performed using enzymes, such as restriction enzymes, ligases, *etc.,* known in the art, but is not limited thereto.

The microorganism of the present invention may be a microorganism in which Ku70 or Ku80 protein activity is inactivated or activity of URA3 gene is inactivated.

As used herein, the term "Ku70" or "Ku80" refers to a Ku protein, and Ku70 and Ku80 form a Ku heterodimer, which binds to the ends of a DNA double-strand break, and is involved in DNA repair via non-homologous end joining (NHEJ). In the present invention, to increase the efficiency of homologous recombination of the retinol biosynthetic genes, a microorganism was used in which one of Ku70/Ku80 genes having a function of inhibiting random insertion was deleted.

As used herein, the term "URA3 gene" refers to a gene encoding orotidine 5'-phosphate decarboxylase (ODCase) in a pyrimidine synthetic pathway of a yeast. Further, URA3+ yeast is characterized in that it may proliferate in a medium to which uracil or uridine is not added, but it does not proliferate in a medium containing 5-fluoroorotic acid (5-FOA), which is a pyrimidine analog. URA3 gene may be used as a positive or negative marker by employing the above characteristics.

According to one exemplary embodiment of the present invention, to examine whether or not the retinol biosynthetic genes were introduced, a URA3 gene-deleted yeast was prepared. A method of introducing the URA3 gene together, when the retinol biosynthetic genes were introduced, was used to select a strain in which the retinol biosynthetic genes were introduced in a medium to which uracil or uridine was not added (Example 1-1).

As used herein, the term "inactivation" refers to a case where the activity of an enzyme protein originally possessed by a microorganism is weakened, as compared to the endogenous activity thereof or the activity before modification; a case where no protein is expressed at all; or a case where the protein is expressed but exhibits no activity. The inactivation is a concept that includes a case where the activity of an enzyme itself is weakened or eliminated, as compared to the activity of the enzyme originally possessed by a microorganism, due to a modification in the polynucleotide encoding the enzyme, *etc.;* a case where the level of overall enzyme activity within a cell is reduced or eliminated, as compared to the wild-type microorganism, due to inhibition of expression of the gene encoding the enzyme, or inhibition of translation, *etc.;* a case where a part or the entirety of the gene encoding the enzyme is deleted; and a combination thereof, but is not limited thereto. In other words, the microorganism in which activity of the enzyme protein is inactivated refers to a microorganism in which the activity of the enzyme protein is reduced or eliminated, as compared to the natural wild-type microorganism or the non-modified microorganism.

The inactivation of the enzyme activity may be achieved by way of various methods well known in the art. Examples of the methods include 1) a method of deleting a part or the entirety of the gene encoding the enzyme on the chromosome; 2) a method of modifying an expression regulatory sequence such that the expression of the gene encoding the protein on the chromosome is reduced; 3) a method of modifying a gene sequence encoding the protein on the chromosome such that the activity of the protein is removed or weakened; 4) a method of introducing an antisense oligonucleotide (e.g., antisense RNA) that binds complementarily to the transcript of the gene encoding the protein on the chromosome; 5) a method of adding a sequence complementary to the Shine- Dalgarno sequence of the gene encoding the protein on the chromosome upstream of the Shine-Dalgarno sequence to form a secondary structure, thereby making the adhesion of ribosome impossible; and 6) a method of reverse transcription engineering (RTE), which adds a promoter, which is to be reverse-transcribed, to the 3'-end of the open reading frame (ORF) of the polynucleotide sequence encoding the protein, or a combination thereof, but are not limited particularly thereto.

The method of deleting a part or the entirety of the gene encoding the enzyme on the chromosome may be performed by replacing the polynucleotide encoding the endogenous target protein within the chromosome with a polynucleotide having a partially deleted nucleic acid sequence, or a marker gene through a vector for chromosomal insertion into a microorganism. As an example of the method of deleting a part or the entirety of the polynucleotide, a method of deleting the polynucleotide by homologous recombination may be used, but is not limited thereto.

The method of modifying the expression regulatory sequence may be performed by inducing a modification in the expression regulatory sequence through deletion, insertion, non-conservative or conservative substitution, or a combination thereof so as to further weaken the activity of the expression regulatory sequence; or by replacing the sequence with a nucleic acid sequence having a weaker activity. The expression regulatory sequence may include a promoter, an operator sequence, a sequence encoding a ribosome binding site, and a sequence for regulating the termination of transcription and translation, but is not limited thereto.

The method of modifying the gene sequence on the chromosome may be performed by inducing a modification in the gene sequence through deletion, insertion, non- conservative or conservative substitution, or a combination thereof so as to further weaken the activity of the enzyme; or by replacing the sequence with a gene sequence modified to have a weaker activity or a gene sequence modified to have no activity at all, but is not limited thereto.

As used herein, the term "part", although it may vary depending on the kinds of polynucleotide, may specifically refer to 1 to 300 nucleotides, more specifically 1 to 100 nucleotides, and even more specifically 1 to 50 nucleotides, but is not particularly limited thereto.

The microorganism for producing retinol may be a microorganism in which geranylgeranyl diphosphate synthase (GGPP synthase, crtE), phytoene synthase (crtYB), and desaturase (crtI) protein activities are enhanced, and may be a microorganism capable of producing *beta*-carotene and having more improved ability to produce retinol by further enhancing *beta*-carotene 15,15'-monooxygenase and retinol dehydrogenase protein activities.

The microorganism of the present invention is not limited, as long as it is a microorganism having an ability to produce retinol, and specifically, the microorganism may be *Yarrowia lipolytica* or *Saccharomyces cerevisiae.*

Another aspect of the present invention provides a method of producing retinol, the method including the step of culturing the microorganism of the present invention in a culture medium.

The "microorganism" and "retinol" are the same as described above.

As used herein, the term "culturing" refers to growing the microorganism in an appropriately controlled environment. The culture process of the present invention may be achieved according to appropriate medium and culture conditions known in the art. The culture process may be easily adjusted for use by those skilled in the art according to the microorganism to be selected. Although not particularly limited thereto, the step of culturing the microorganism may be performed by a known batch culture method, continuous culture method, fed-batch culture method, *etc.* The medium used for culturing the microorganism of the present disclosure and other culture conditions are not particularly limited, but any medium commonly used for culturing the microorganism may be used. Specifically, the microorganism of the present disclosure may be cultured in a common medium containing suitable carbon sources, nitrogen sources, phosphorous sources, inorganic compounds, amino acids, and/or vitamins, *etc.,* under aerobic conditions while controlling temperature, pH, *etc.*

The pH in the step of culturing may be adjusted using a basic compound (e.g., sodium hydroxide, potassium hydroxide, or ammonia) or an acidic compound (e.g., phosphoric acid or sulfuric acid), and specifically, the pH may be 5.5 to 7.5, 5.5 to 7.0, 6.0 to 7.5, 6.0 to 7.0, 6.5 to 7.5, or 6.5 to 7.0, and more specifically, the pH in the step of culturing may be 6.9, but is not limited thereto.

Further, oxygen or oxygen-containing gas may be injected into the culture in order to maintain an aerobic state of the culture; or no gas may be injected, or nitrogen gas, hydrogen gas, or carbon dioxide gas may be injected to maintain an anaerobic or microaerobic state. The aeration rate in the step of culturing may be 0.2 vvm to 1.5 vvm, 0.2 vvm to 1.3 vvm, 0.2 vvm to 1.1 vvm, 0.5 vvm to 1.5 vvm, 0.5 vvm to 1.3 vvm, 0.5 vvm to 1.1 vvm, 0.8 vvm to 1.5 vvm, 0.8 vvm to 1.3 vvm, 0.8 vvm to 1.1 vvm, and more specifically, the aeration rate in the step of culturing may be 0.9 vvm, but is not limited thereto.

Further, the culture temperature may be maintained at 20°C to 45°C, or 25°C to 40°C, specifically at 27°C to 31°C, 28°C to 31°C, 29°C to 31°C, or 30°C to 31°C, and more specifically at 30.2°C, but is not limited thereto.

Further, the reactor operating rpm in the step of culturing may be 50 rpm to 300 rpm, 50 rpm to 250 rpm, 100 rpm to 300 rpm, 100 rpm to 250 rpm, 150 rpm to 300 rpm, 150 rpm to 250 rpm, 200 rpm to 300 rpm, or 200 rpm to 250 rpm, and more specifically 249.9 rpm, but is not limited thereto.

As used herein, the term "medium" refers to a culture medium for culturing the microorganism of the present invention and/or a product obtained after culturing the same. The medium is a concept that includes both a form containing the microorganism and a form in which the microorganism is removed by centrifugation, filtration, *etc.* from the culture solution containing the microorganism.

In the culture medium used for culturing the microorganism for producing retinol of the present invention, as a carbon source, sugars and carbohydrates (e.g., glucose, sucrose, lactose, fructose, maltose, molasses, starch, and cellulose), oils and fats *(e.g.,* soybean oil, sunflower seed oil, peanut oil, and coconut oil), fatty acids *(e.g.,* palmitic acid, stearic acid, and linoleic acid), alcohols *(e.g.,* glycerol and ethanol), organic acids *(e.g.,* acetic acid), *etc.* may be used alone or in combination, but the carbon source is not limited thereto. As a nitrogen source, nitrogen-containing organic compounds (e.g., peptone, yeast extract, meat gravy, malt extract, corn steep liquor, soybean flour, and urea) or inorganic compounds (e.g., ammonium sulfate, ammonium chloride, ammonium phosphate, ammonium carbonate, and ammonium nitrate), *etc.* may be used alone or in combination, but the nitrogen source is not limited thereto. As a phosphorus source, potassium dihydrogen phosphate, dipotassium hydrogen phosphate, corresponding sodium-containing salts thereof, *etc.* may be used alone or in combination, but the phosphorus source is not limited thereto. Further, essential growth-promoting materials such as other metal salts (e.g., magnesium sulfate or iron sulfate), amino acids, vitamins, *etc.* may be included in the medium.

The culture medium used for culturing the microorganism for producing retinol of the present invention may include one or more nutrients selected from the group consisting of yeast extract, peptone, soybean, and glucose.

The yeast extract may be included in an appropriate amount in the culture medium for the microorganism for producing retinol of the present invention, specifically in an amount of 1 part by weight to 4 parts by weight, 1.5 parts by weight to 4 parts by weight, 2 parts by weight to 4 parts by weight, 2.5 parts by weight to 4 parts by weight, 1 part by weight to 3.5 parts by weight, 1.5 parts by weight to 3.5 parts by weight, 2 parts by weight to 3.5 parts by weight, or 2.5 parts by weight to 3.5 parts by weight, and more specifically in an amount of 3 parts by weight, based on the total 100 parts by weight of the culture medium, but is not limited thereto.

Further, the peptone may be included in an appropriate amount in the culture medium for the microorganism for producing retinol of the present invention, specifically in an amount of 0.5 parts by weight to 4 parts by weight, 1 part by weight to 4 parts by weight, 1.5 parts by weight to 4 parts by weight, 2 parts by weight to 4 parts by weight, 2.5 parts by weight to 4 parts by weight, 0.5 parts by weight to 4 parts by weight, 1 part by weight to 3.5 parts by weight, 1.5 parts by weight to 3.5 parts by weight, 2 parts by weight to 3.5 parts by weight, or 2.5 parts by weight to 3.5 parts by weight, and more specifically in an amount of 3 parts by weight, based on the total 100 parts by weight of the culture medium, but is not limited thereto.

Further, the soybean may be included in an appropriate amount in the culture medium for the microorganism for producing retinol of the present invention, specifically in an amount of 0.5 parts by weight to 4 parts by weight, 1 part by weight to 4 parts by weight, 1.5 parts by weight to 4 parts by weight, 2 parts by weight to 4 parts by weight, 2.5 parts by weight to 4 parts by weight, 0.5 parts by weight to 4 parts by weight, 1 part by weight to 3.5 parts by weight, 1.5 parts by weight to 3.5 parts by weight, 2 parts by weight to 3.5 parts by weight, or 2.5 parts by weight to 3.5 parts by weight, and more specifically in an amount of 3 parts by weight, based on the total 100 parts by weight of the culture medium, but is not limited thereto.

Further, the glucose may be included in an appropriate amount in the culture medium for the microorganism for producing retinol of the present invention, specifically in an amount of 1 part by weight to 3 parts by weight, 1 part by weight to 2.5 parts by weight, 1.5 parts by weight to 3 parts by weight, or 1.5 parts by weight to 2.5 parts by weight, and more specifically in an amount of 2 parts by weight, based on the total 100 parts by weight of the culture medium, but is not limited thereto.

The retinol produced by the culture may be secreted into the medium or may remain in the cells.

Further, the method of producing retinol of the present invention may be a method of using a multi-copy plasmid of the microorganism. Specifically, in one exemplary embodiment, it was confirmed that the retinol production was increased about 3-fold in a strain transformed with the multi-copy plasmid, as compared with a strain transformed with a single-copy plasmid (Example 3-2).

Further, the method of producing retinol of the present invention may further include the step of recovering retinol from the cultured microorganism or medium. In the step of recovering retinol produced in the culturing step of the present invention, the desired retinol may be collected from the microorganism for producing retinol and the culture solution thereof using an appropriate method known in the art according to the culture method. For example, lyophilization, centrifugation, filtration, anion-exchange chromatography, crystallization, HPLC, *etc.* may be used, and the desired retinol may be recovered from the cultured microorganism or the medium using an appropriate method known in the art. The step of recovering retinol may further include a separation process and/or a purification step.

Hereinafter, the present invention will be described in more detail with reference to Examples. However, these Examples are for illustrative purposes only, and the scope of the present invention is not intended to be limited by these Examples.

### Example 1: Selection of strain for producing retinol and gene synthesis through codon optimization

### Example 1-1: Preparation of Saccharomyces cerevisiae platform strain for producing retinol

*Saccharomyces cerevisiae,* which is a yeast strain widely used in recombinant protein production, was selected as a strain for producing retinol.

Next, to prepare the strain for producing retinol, high-efficiency gene manipulation in the genome of a *Saccharomyces cerevisiae* strain is required, and to this end, it is necessary to establish a high-efficiency gene manipulation system capable of repeatedly integrating or removing a specific gene group in the genome, and a system capable of performing a homologous recombination of the integrated gene within the genome with high efficiency.

Therefore, a *Saccharomyces cerevisiae* platform strain was prepared in which the above system was established.

In detail, to increase efficiency of homologous recombination, one of Ku70/Ku80 genes having the function of inhibiting random insertion should be removed, and to use URA3 as an auxotrophic selection marker for confirming foreign gene integration, URA3 gene in the genome should be removed. Accordingly, a *Saccharomyces cerevisiae* platform strain for producing retinol was prepared in which URA3 and Ku70 were removed. This strain was used in the following experiments.

### Example 1-2: Design of Saccharomyces cerevisiae customized high-efficiency retinol production metabolic pathways and gene synthesis

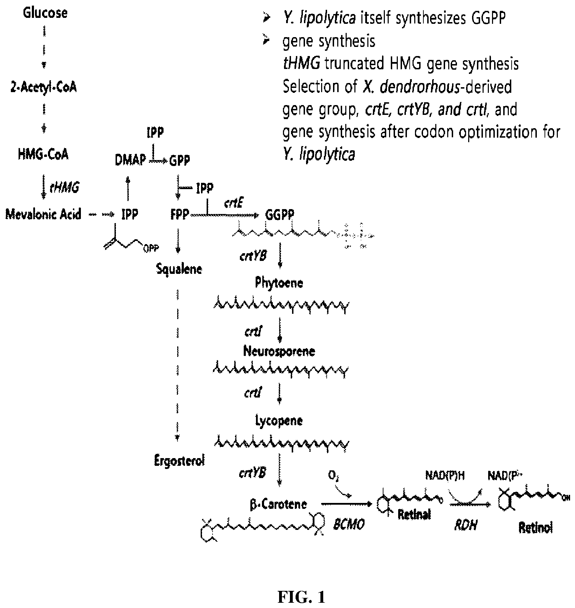

As confirmed in FIG. 1, metabolic pathways from GGPP production to retinol production (GGPP → phytoene → neurosporene → lycopene → β-carotene → retinal → retinol) were designed.

In detail, geranylgeranyl diphosphate synthase (GGPP synthase), phytoene synthase, and desaturase-encoding nucleotide sequences (crtE, crtI, and crtYB, respectively), which are *Xanthophyllomyces dendrorhous-*derived beta-carotene biosynthetic genes, were codon- optimized for *Saccharomyces cerevisiae,* and then each gene was synthesized; a *beta*-carotene 15,15'-monooxygenase (β-carotene 15,15'-monooxygenase)-encoding nucleotide sequence (BMCO-blh or BMCO-SR), which is *Halobacterium* sp. NRC-1- or marine bacterium 66A03-derived retinal biosynthetic gene, was codon-optimized for *Saccharomyces cerevisiae,* and then each gene was synthesized; and a retinol dehydrogenase-encoding nucleotide sequence (ybbO), which is a retinol biosynthetic gene, was codon-optimized for *Saccharomyces cerevisiae,* and then the gene was synthesized.

The nucleotide sequences encoding crtE, crtYB, crtI, BMCO-blh, BMCO-SR, and ybbO are represented by SEQ ID NO: 1, SEQ ID NO: 2, SEQ ID NO: 3, SEQ ID NO: 4, SEQ ID NO: 5, and SEQ ID NO: 6, respectively.

### Example 2: Preparation of strain for producing retinol

### Example 2-1: Preparation of expression vector for S. cerevisiae for producing retinol

To stably express crtE, crtI, crtYB, BMCO-blh, BMCO-SR, and ybbO gene nucleotide sequences synthesized in Example 1-2 in the *Saccharomyces cerevisiae* platform strain for producing retinol, an expression vector was prepared in which a GPD promoter and a CYC1 terminator were included and a ura marker system for repeated use of the vector system was applied.

In detail, crtE, crtI, crtYB, BMCO-blh, BMCO-SR, and ybbO gene-encoding nucleotide sequences synthesized in Example 1-2 were inserted into the expression vector, into which the GPD promoter, the CYC1 terminator, and the URA Blaster cassette were introduced, using EcRI and XhoI restriction enzymes within the multi cloning site, respectively. pBCA_crtE, pBCA_crtI, pBCA_crtYB, pRET_BMCO-blh, pRET_BMCO-SR, and pRET_ybbO vectors, into which each gene sequence was inserted, were prepared, respectively.

### Example 2-2: Integration of retinol precursor biosynthetic genes

As integration sites in the genome of S. *cerevisiae,* a YPL062w gene known to increase production of mevalonate, which is a precursor of retinol, when removed, and LPP1 and DPP1 genes producing farnesol, which are involved in farnesyl pyrophosphate (FPP) depletion, were selected in order to increase supply of the retinol precursor.

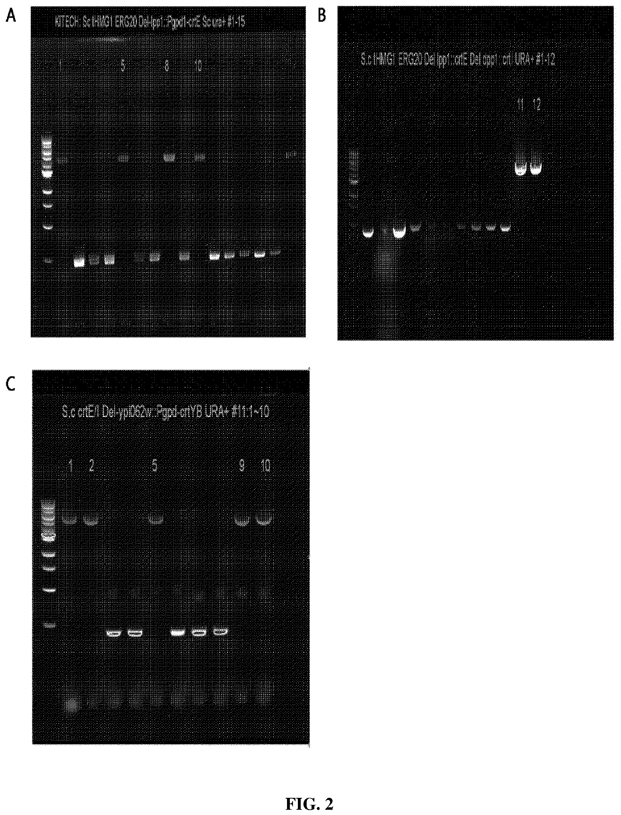

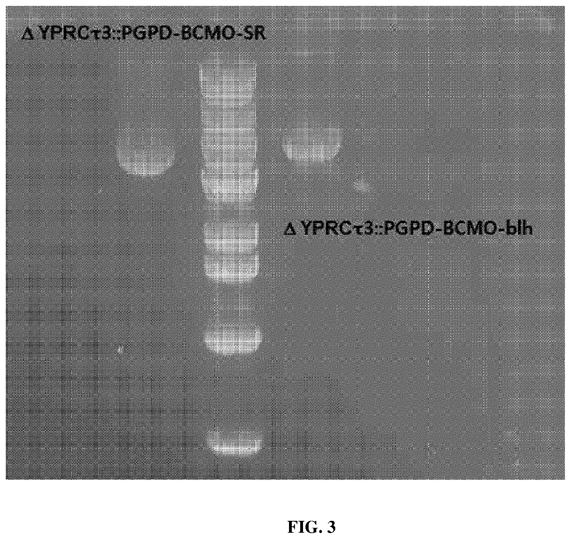

In detail, the *Saccharomyces cerevisiae* platform strain for producing retinol, which was prepared in Example 1-1, was transformed with the pBCA_crtE vector prepared in Example 2-1, and then it was examined through colony PCR whether or not the crtE gene was integrated at the correct site in the strain (FIG. 2A). When crtE gene was integrated at the correct site, bands of 4,000 bp were observed, as in lanes 1, 5, 8, and 10 of FIG. 3A. The ura marker was removed from the strain, in which the integration was confirmed, through ura pop-out, and then used in the subsequent crtI gene integration.

The method above was used to integrate crtI gene and crtYB gene sequentially, and the integration of the genes was examined (FIGS. 2B and 2C). When crtI gene is integrated at the correct site, bands appear around 4,000 bp, as in lanes 11 and 12 of FIG. 2B. When crtYB gene is integrated at the correct site, bands appear around 6,000 bp, as in lanes 1, 2, 5, 9, and 10 of FIG. 2C. As described, the strain, in which crtE, crtI, and crtYB genes which are biosynthetic genes of *beta*-carotene as a retinol precursor were integrated, was prepared. In the following experiment, the strain *(S. cerevisiae* CEN.PK2-1D Δleu2::PTEF1-ERG20 Δhis3::PCCW12-tHMG1 Δlpp1::PGPD-crtE Δdpp1::PGPD-crtI Δypl062w::PGPD-crtYB) was used as a basic strain to prepare a strain for producing retinol.

### Example 2-3: Integration of retinol biosynthetic genes

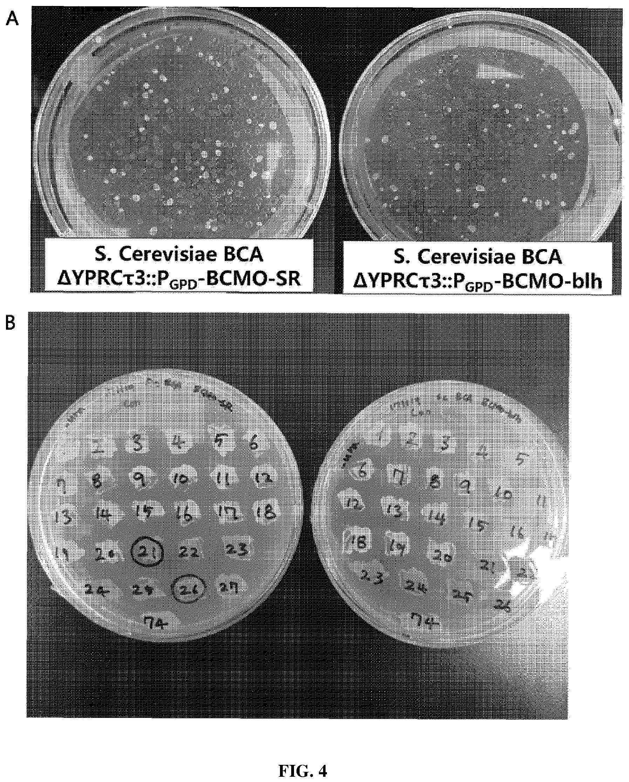

The *Saccharomyces cerevisiae* strain prepared in Example 2-2, in which the retinol precursor biosynthetic genes were integrated, was transformed with the pBCA-BCMO vector prepared in 2-1, and then it was examined through PCR and colony formation whether or not each of BMCO-blh or BMCO-SR genes was integrated at the correction site in the strain (FIGS. 3 and 4).

In detail, pBCA-BCMO vector and pUC-3Myc URA3-PGPD vector were treated with EcoRI/XhoI restriction enzymes to digest BCMO, followed by electrophoresis on a 1% agarose gel. The digested BCMO was purified, and the purified BCMO was ligated with the pUC-3Myc URA3-PGPD vector using EcoRI/XhoI restriction enzymes to prepare a pUC-3Myc URA3-PGPD-BCMO vector. BCMO was digested again using EcoRI/XhoI restriction enzymes. Thereafter, the BCMO gene was integrated into the YPPCτ3 gene site using primers for an integration cassette, consisting of nucleotide sequences in Table 1 below.

**[Table 1]**

| Primer sequences for BCMO gene integration cassette | |
|---|---|
| Forward | ATC GTC CTT GTA TGG AAG TAT CAA AGG GGA CGT TCT TCA CCT CCT TGG AAC CAG TCA CGA CGT TGT AAA A (SEQ ID NO: 13) |
| Reverse | AAT GAT TTA CAA TCT AGT CGC AAA AAC AAG TAC AGT GCT GAC GTC CCA TCA GGT TTC CCG ACT GGA AAG C (SEQ ID NO: 14) |

As a result, as shown in FIG. 3, PCR results confirmed that a vector for PGPD-BCMO URA3 integration was prepared in which BCMO-SR or BCMO-blh gene was integrated. Subsequently, as shown in FIGS. 4A and 4B, it was confirmed through colony formation and master colony acquisition that BMCO-blh or BMCO-SR gene was successfully transformed into the *Saccharomyces cerevisiae* YPRCτ3 gene site by the prepared integration cassette. Subsequently, as shown in FIGS. 4C and 4D, PCR of the BCMO-integrated master colony was performed, and as a result, when BCMO-SR gene was integrated, a band appeared around 5,124 bp, as in lane 21, and when BCMO-blh gene was integrated, a band appeared around 5,124 bp, as in lanes 11 and 14. As described, a BCMO gene-introduced strain was prepared.

Finally, to prepare a strain for producing retinol, a strain for producing retinol was prepared in which the ybbO gene encoding retinol dehydrogenase was introduced into the prepared strain.

In detail, in the same manner as the method of introducing BCMO gene, a vector for pUC57-3Myc URA3-PGPD-ybbO integration was prepared. Thereafter, the ybbO gene was integrated into the YPRCdelta15 gene site using primers for an integration cassette, consisting of nucleotide sequences in Table 2 below.

**[Table 2]**

| Primer sequences for ybbO gene integration cassette | |
|---|---|
| Forw ard | AAATCCGAACAACAGAGCATAGGGTTTCGCAAACAAACTTAAAT ATATGCaggtttcccgactggaaag (SEQ ID NO: 15) |
| Rever se | GTATAATCTGTATACATAATATTATAGGCTTTACCAACAATGGAAT TTCGccagtcacgacgttgtaaaa (SEQ ID NO: 16) |

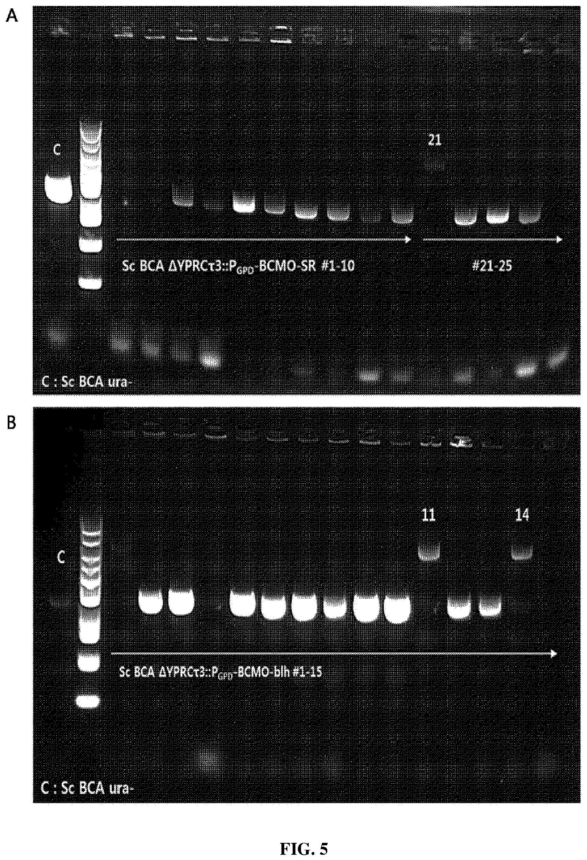

As a result, as shown in FIG. 5, PCR of the ybbO-integrated master colony was performed, and as a result, when ybbO gene was integrated, a band appeared around 3,600 bp, as in lane 11. Finally, a strain for producing retinol was prepared in which crtE, crtYB, crtI, BMCO-blh, BMCO-SR, and ybbO genes were integrated.

### Example 3: Examination of retinol production of strain for producing retinol

### Example 3-1: Examination of retinol production of strain for producing retinol

To examine retinol production by the strain for producing retinol, HPLC/UV chromatography and ESI-mass spectrometry were performed. In detail, chromatographic analysis was performed using an analytical scale (dimensions: 300 mm × 4 mm). An ODS C18 guard column (4 mm × 3 mm) preceded a running column.

Chromatographic conditions were as follows: mobile phase A: acetonitrile/H₂O/glacial acetic acid = 90/10/2; mobile phase B: acetonitrile/methanol = 90/10; mobile phase C: 100% THF. All mobile phases were filtered through nylon membrane filters with 0.45 µm pore size before use. Over a total run time of 26 min, the following gradient program was applied at a constant flow rate (1 mL/min) and a constant column temperature (22°C): 0-4.5 min 100% A; 4.5-6.5 min 100% B; 6.5-12 min 60% B, 40% C; 12-15 min 60% B, 40% C; 15-20 min 100% B; 20-26 min 100% A.

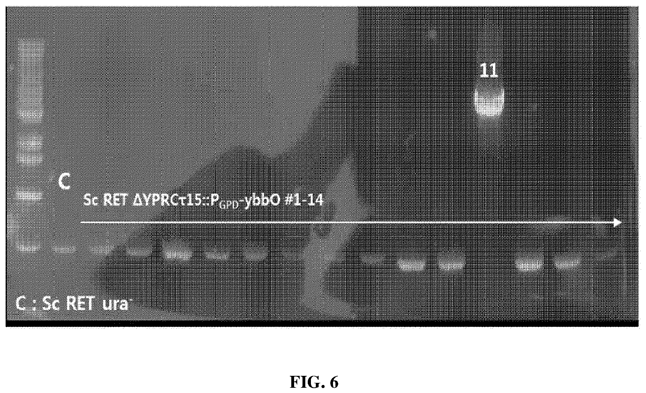

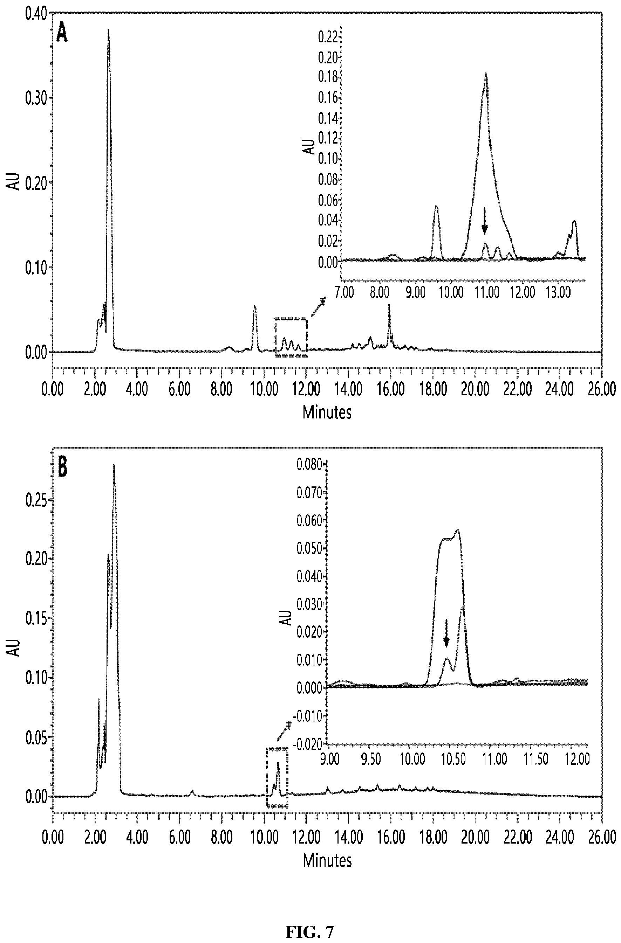

As a result, as shown in FIG. 6, a retinal production peak and a retinol production peak of the colony formed by culturing the strain for producing retinol were observed. In detail, as a result of HPLC/UV chromatography (325 nm), the retinal production peak was observed at 10.47 min (FIG. 6A), and the retinol production peak was observed at 10.97 min. Further, as shown in FIG. 7, unlike CEN.PK wild-type strain (FIG. 7A), the strain for producing retinol of the present invention showed the retinol production peak at 303.2318 m/z (FIG. 7B).

These results indicate that the strain for producing retinol prepared in Example 2 is able to produce retinol.

### Example 3-2: Examination of effect of increasing retinol production by multi-copy plasmid

The effect of increasing the retinol production of the strain for producing retinol was examined when the strain transformed with a multi-copy plasmid was cultured.

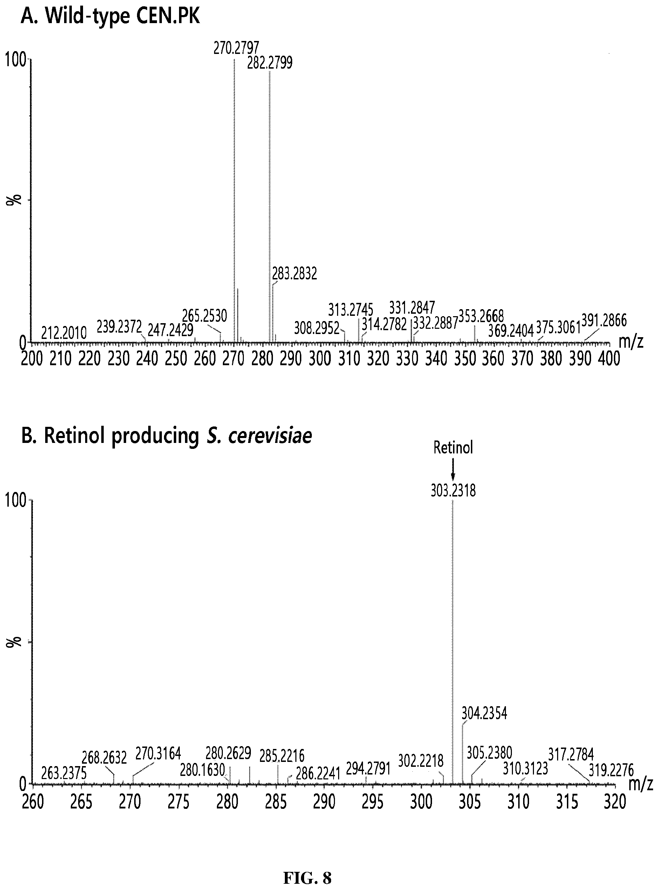

In detail, as shown in FIG. 8, when the strain was cultured by introducing the single-copy BCMO-SR gene using a pUC57 vector, it produced 169.16 µg/L of retinol. In contrast, when the strain was cultured by introducing the multi-copy BCMO-SR gene using a p426 vector, it produced 491.09 µg/L of retinol. That is, the strain transformed with the multi-copy showed an about 3-fold increase in the retinol production, as compared with the strain transformed with the single copy. These results indicate that when the microorganism transformed with the multi-copy plasmid is cultured, the retinol production may be remarkably increased.

Descriptions of contents which may be sufficiently recognized and inferred by those skilled in the art will be omitted in the present specification. In addition to the specific exemplary embodiments described in the present specification, more various modifications may be made without changing the technical spirit or essential configuration of the present invention. Accordingly, the present invention may be implemented in a manner different from those specifically described and illustrated in the present specification, which may be understood by those skilled in the art.

### Effect of the invention

A microorganism of the present invention may have an improved ability to produce retinol, and thus it may be efficiently used in producing retinol. Based on a method of producing retinol, the method including a step of culturing the microorganism, the retinol production efficiency may be improved.

## Claims

1. A microorganism for producing retinol, in which geranylgeranyl diphosphate synthase (GGPP synthase, crtE), phytoene synthase (crtYB), desaturase (crtI), *beta-* carotene 15,15'-monooxygenase (β-carotene 15,15'-monooxygenase, BCMO), and retinol dehydrogenase (ybbO) protein activities are enhanced.

2. The microorganism for producing retinol of claim 1, wherein the geranylgeranyl diphosphate synthase (GGPP synthase, crtE), phytoene synthase (crtYB), and desaturase (crtI) are derived from *Xanthophyllomyces dendrorhous.*

3. The microorganism for producing retinol of claim 1, wherein the *beta*-carotene 15,15'-monooxygenase (β-carotene 15,15'-monooxygenase, BCMO), and retinol dehydrogenase (ybbO) are derived from a *Halobacterium* sp. NRC-1, marine bacterium 66A03, or *Escherichia* sp. microorganism.

4. The microorganism for producing retinol of claim 1, wherein a gene of the geranylgeranyl diphosphate synthase (GGPP synthase, crtE) includes a nucleotide sequence of SEQ ID NO: 1; a gene of the phytoene synthase (crtYB) includes a nucleotide sequence of SEQ ID NO: 2; a gene of the desaturase (crtI) includes a nucleotide sequence of SEQ ID NO: 3; a gene of the *beta*-carotene 15,15'-monooxygenase (β-carotene 15,15'-monooxygenase, BCMO) includes a nucleotide sequence of SEQ ID NO: 4 or SEQ ID NO: 5; and a gene of the retinol dehydrogenase (ybbO) includes a nucleotide sequence of SEQ ID NO: 6.

5. The microorganism for producing retinol of claim 1, wherein the microorganism is *Yarrowia lipolytica* or *Saccharomyces cerevisiae.*

6. A method of producing retinol, the method comprising the step of culturing the microorganism of any one of claims 1 to 5 in a culture medium.

7. The method of producing retinol of claim 6, wherein the culture medium includes one or more nutrients selected from the group consisting of yeast extract, peptone, soybean, and glucose.

8. The method of producing retinol of claim 6, further comprising the step of recovering retinol from the cultured microorganism or medium.


<110>     KOREA INSTITUTE OF INDUSTRIAL TECHNOLOGY

<120>     A microorganism for producing a bio-retinol and a method for
          preparing a bio-retinol using the same

<130>     OPA19197-PCT

<150>     KR 10-2018-0152617
<151>     2018-11-30

<160>     16

<170>     KoPatentIn 3.0

<210>     1
<211>     2022
<212>     DNA
<213>     GGPP synthase, crtE

<400>     1
atgacggcat tggcatacta ccagatccac ctcatctaca ccctccccat cctcggtctc        60

ctcggtctgc ttacaagccc catcctcaca aagttcgaca tctacaagat ctcgatcctc       120

gtgttcatcg cgttctccgc cacgacgccc tgggacagct ggatcatccg aaacggcgca       180

tggacgtacc ctagcgcaga atctggtcaa ggtgttttcg gcacgttcct ggacgttccc       240

tacgaggagt acgcattctt cgtgatccag acggtgatca ctggcctggt ctacgtcctc       300

gcaacacgac acctcctccc atctctggca ttgcctaaga cacgttcctc tgctctctct       360

ctggctctca aggcactcat ccctttgcct atcatctacc tgttcaccgc ccacccatcc       420

ccatctccag acccattggt caccgaccat tacttctaca tgcgggctct gtccctcctc       480

atcacccctc ctactatgct gttggctgct ttgtccggtg agtacgcttt cgactggaag       540

tcgggaagag ctaagtccac catcgctgct atcatgatcc ccaccgtgta cctcatctgg       600

gtggactacg tcgctgtggg acaagactct tggtcgatta acgacgagaa gattgtcggc       660

tggcgactgg gaggagtcct gcctattgag gaggctatgt tcttcctgct caccaacctg       720

atgattgtgc tcggcctctc cgcctgcgac catacacagg ctctctacct gttgcacggt       780

agaaccattt acggcaacaa gaagatgccg tcgtccttcc ccctgattac cccccctgtg       840

ctgtctctct tcttcagctc ccgaccttac tcgtcccagc ctaagcgaga cttggagttg       900

gctgtgaagc tgctggagga gaagtctcgt agcttcttcg tcgcttccgc tggattccct       960

tctgaggtca gagagcgact cgttggtttg tacgcgtttt gccgagtgac cgacgacctc      1020

attgactcgc ctgaggttag ctctaaccct cacgctacaa ttgacatggt gtccgacttt      1080

ctgaccctgc tctttggacc gccgctgcac ccttctcagc ctgacaagat tctgtcctcg      1140

cctctcctcc ctccttctca cccctctaga cccactggaa tgtacccctt gcccccacca      1200

ccgtctttgt ctcccgctga attggttcaa tttcttacag agcgtgttcc cgttcaatac      1260

cattttgcct ttcgtctgct ggccaagctg cagggtctga ttccgcgtta cccgctggat      1320

gagctgcttc ggggttatac caccgatctg atttttcccc tgtccaccga ggctgttcag 1380

gcgagaaaga cccccattga gaccaccgcc gatcttcttg actacggtct ttgtgtggcc 1440

ggatctgttg ccgaacttct ggtttatgtg tcctgggcgt ctgcgccctc tcaggttccc 1500

gcgactattg aagagagaga ggccgttctt gtggcctcta gagaaatggg aaccgccctt 1560

cagcttgtga atattgcccg agacattaag ggcgatgcca ctgagggacg attttatctg 1620

cccctgagct tttttggact gcgggatgag tcgaagctgg ccattcccac tgattggact 1680

gagccgcgac cccaggattt tgataagctg ctgtcgctga gcccctcgtc gactctgccc 1740

agctccaatg cctcggaatc ctttcgattt gagtggaaga cttattcgct gccccttgtg 1800

gcctatgccg aggatcttgc caaacattcg tataaaggca ttgatcgact tcccactgaa 1860

gtccaggccg gcatgcgagc cgcctgtgcc agctatcttc ttattggacg ggaaattaaa 1920

gtcgtctgga aaggcgatgt gggagaacga cggactgtgg ccggctggcg gcgagtccga 1980

aaagtccttt ccgtcgtcat gtcgggctgg gaaggccagt aa 2022

<210> 2
<211> 1131
<212> DNA
<213> Phytoene synthase, crtYB

<400> 2
atggactacg caaacatcct caccgcaatc ccattggagt tcaccccaca ggatgatatc 60

gtgctgttgg agccttacca ctacctcggt aagaaccctg gtaaggagat ccgttctcag 120

ctcatcgagg cattcaacta ctggctggat gtcaagaagg aggacctgga ggtcatccag 180

aacgtcgttg gaatgttgca cacggcttct ttgctcatgg acgatgtgga ggattcttcg 240

gtgttgcgta gaggttctcc tgttgctcac ctcatctacg gtatccctca gacaatcaac 300

accgctaact acgtgtactt cctggcgtac caggagatct tcaagctgcg acctacgcct 360

atccccatgc ccgttatccc cccgtcttct gcttctttgc aatcttctgt ttcttccgct 420

tcttcttcct ccagcgctag ctccgaaaac ggtggaacaa gcacacccaa ttcccaaatc 480

cccttctcca aggatacata cctcgataag gtgattacgg acgagatgct ctccctgcac 540

agaggacagg gacttgagct tttctggaga gactcgctta cttgcccgtc ggaagaagag 600

tacgttaaga tggtgctggg aaagaccgga ggccttttcc gaattgccgt tcgacttatg 660

atggccaagt ccgagtgtga cattgacttc gtccagcttg tcaacctgat ttccatttac 720

tttcagattc gagacgacta catgaacctg cagtcgtcgg agtatgccca caacaagaac 780

tttgccgagg acctgaccga gggcaagttt tcctttccca ccattcactc gattcatacc 840

aaccccagct cgcggctggt gattaacacc ctgcagaaga agtccacctc gcccgagatt 900

ctccatcatt gcgtcaacta tatgcggact gagactcatt cgtttgagta tactcgagag 960

gtgctgaata ctctgtccgg cgcgctggag cgtgagctcg gccgacttca agaagaattt 1020

gccgaagcca atagccggat ggacctgggc gacgtcgaaa gcgaaggccg aactggcaaa 1080

aatgtgaaac tggaagccat tctcaaaaaa ctcgccgaca ttccgctcta a 1131

<210> 3
<211> 1749
<212> DNA
<213> desaturase, crtI

<400> 3
atgggtaagg agcaggatca ggataagccg acagctatca tcgtcggttg cggtatcgga 60

ggaatcgcaa cagctgcacg tctcgcaaag gagggtttcc aagttacggt tttcgagaag 120

aacgactaca gcggcggtcg ttgcagcttg atcgagcgtg atggttaccg tttcgaccag 180

ggtccatctt tgctgttgct gccagatctc ttcaagcaga ccttcgaaga cctgggtgag 240

aagatggagg actgggtgga tctgatcaag tgcgagccaa actacgtgtg ccacttccac 300

gacgaggaga cattcacgct cagcactgat atggctctcc tcaagcgaga ggttgagcgt 360

ttcgagggta aggacggttt cgatcggttc ctctctttca tccaggaggc acaccggcat 420

tacgagttgg cagttgtgca cgttctccag aagaacttcc ctggtttcgc agctttcctc 480

cgactccagt tcatcggtca gatcctggct ctccaccctt tcgaatctat ctggacccga 540

gtttgtcgat acttcaagac cgaccgactg cggcgggttt tctctttcgc tgtgatgtac 600

atgggccagt ctccatactc cgctcctggt acttactcgc tcctccagta cactgagctc 660

actgagggaa tctggtaccc tagaggagga ttctggcagg ttcctaacac cctcctccag 720

atcgttaagc gaaacaaccc gtctgctaag ttcaacttca acgcgccggt ctcccaggtt 780

ttgctgtcgc ctgctaagga tcgagctact ggagttcgac tggagtctgg agaggagcat 840

cacgctgatg tcgtcatcgt gaacgctgat ttggtgtacg cttctgagca cctcatccct 900

gatgacgctc ggaacaagat cggacagttg ggagaggtga agagatcttg gtgggcggat 960

ctcgtgggag gaaaaaagtt gaagggatcg tgttcgtccc tgtcttttta ctggtccatg 1020

gatcgaattg tggacggcct gggcggacac aacatttttc tggcggagga ctttaagggc 1080

agctttgaca ccatttttga ggaactgggc ctgcccgccg acccttcttt ttacgtcaac 1140

gtgccctcgc gaattgaccc ctccgccgcc cctgaaggaa aagacgccat tgtcattctg 1200

gtcccttgtg gacatattga cgcctctaac ccccaggact ataacaagct ggtcgcccga 1260

gccagaaaat ttgtgattca caccctgtcc gcgaagctgg gactgcccga ctttgaaaag 1320

atgattgtgg ccgaaaaggt ccacgacgcc ccctcctggg aaaaagaatt taacctgaag 1380

gacggctcca ttctgggcct ggcccataat tttatgcaag tgctgggctt tcgaccgagc 1440

acgcgacatc ccaagtatga caagctgttt tttgtcggcg cctccaccca tcccggaact 1500

ggcgtgccca ttgtccttgc cggcgccaaa cttaccgcca atcaagtcct tgaatcgttt 1560

gacagatccc ccgcccccga ccccaatatg tcgctttccg tgccctatgg caaacccctt 1620

aaatcgaatg gcaccggcat tgactcgcaa gtccaactta agtttatgga cctggaaaga 1680

tgggtgtatc tgcttgtcct tcttattggc gccgtcattg ccagaagcgt gggcgtgctt 1740

gccttttaa 1749

<210> 4
<211> 957
<212> DNA
<213> Halobacterium salinarum

<400> 4
gaggacgccc tgcacgcggt ccatccacgt gaccacggcg acgtgtggca gggtgagcac 60

ggcgatcaac acgaggtaga gtgcggcccc ggattcgagc gtggtcggcg ggttgggtac 120

cgcccaccac agcacgccgc ccaacgcgag cgcggccgcc gtcatcggcg cggcctccac 180

gccgaaccgg gccagcggtc cgaggatgtc gcccgcgcgc agcgacgggt ggaccgagcc 240

gtcgacggcg atggcgcgcg cgacgtgccg gaccgagtgc cagacgcaga agtagacgcc 300

gacggcgaac accggcggca cgacgaagaa gaacacccac aacaggaggg tttcggcggc 360

gtcgacgcgc cacgcgccgg gggagtgggt gcggcgtctg cccgccgcga gcaccgcgac 420

ggtcgcggct gcgaacgcga cgcccagcca caggcggacg cgcgggtcga acaccgcgag 480

gtcgccgacg gagccgccga acggcgcggc gaacgcgtcg acgacgctgc ggtaccgctc 540

cgggaacccg agcagcggca cgagcatcgg gaggccaccc cggatcagga ccgtcgccgc 600

gcgccgcggg cgcgtatcga ggtagtcgac gtcgaggaag tcgagcagcg ggtagaggtc 660

gccctgcccc cagtgcagcc acgtgatcgc gacgaacgcg aacgcagcgg gaacgggcgc 720

gaaaaaccac gcggcggcgt agccaccacc gagcacgagg tagaggacgc cgacgaccgc 780

cagccaccgc acggtgacgg tgcccgtgac cgcgcggggc aacgcgaggt agtcgatcgc 840

gccgtgtggc atgccgaaca ccaccgcgct ggcgaccagc ggggcgtacc gtgcggtcgg 900

cgaaagcgac acgccagtga tcgcgggcag ggcagcgatg ctgatcgcca cccagcc 957

<210> 5
<211> 825
<212> DNA
<213> Unknown

<220>
<223> predictet Brp-like protein Blh

<400> 5
atgggcttga tgttaattga ttggtgtgct ttagcattgg ttgtgtttat tggtttgcca 60

catggtgcct tagatgctgc tatttctttt tcaatgattt cttcagcaaa gagaattgct 120

agattagcag gaatactatt aatttacctg ttgttagcaa ccgcattttt tttaatttgg 180

tatcaattac cagcattttc tcttcttatt tttcttttga taagcataat ccattttgga 240

atggctgatt tcaatgcatc cccaagtaaa cttaagtggc ctcatattat tgcacatggc 300

ggcgttgtta ctgtttggtt gccgcttatc caaaaaaatg aagttacgaa gctattttca 360

atattaacaa atggtccaac tcccatttta tgggacatac tattgatatt ttttttatgt 420

tggagcatag gagtatgtct tcatacctat gaaactttac gttctaaaca ttataatatc 480

gcctttgaac ttattggatt aatttttcta gcctggtatg cacccccact cgttactttt 540

gccacatact tctgctttat ccacagcaga cgtcacttta gttttgtttg gaaacagtta 600

cagcatatga gttcaaaaaa aatgatgata ggtagtgcca ttattttatc ttgtacgagc 660

tggttgatag gcggaggaat atattttttc ctcaattcga aaatgattgc cagtgaagct 720

gctttacaaa ctgtctttat tggtcttgca gctttaacag ttcctcacat gatacttatc 780

gactttatat ttagaccaca ctcttccaga attaaaatca aaaat 825

<210> 6
<211> 807
<212> DNA
<213> Escherichia coli

<400> 6
   atgactcata aagcaacgga gatcctgaca ggtaaagtta tgcaaaaatc ggtcttaatt 60
   accggatgtt ccagtggaat tggcctggaa agcgcgctcg aattaaaacg ccagggtttt 120
   catgtgctgg caggttgccg gaaaccggat gatgttgagc gcatgaacag catgggattt 180
   accggcgtgt tgatcgatct ggattcacca gaaagtgttg atcgcgcagc cgacgaggtg 240
   atcgccctga ccgataattg tctgtatggg atctttaaca atgccggatt cggcatgtat 300
   ggcccccttt ccaccatcag ccgtgcgcag atggaacagc agttttccgc caactttttc 360
   ggcgcacacc agctcaccat gcgcctgtta cccgcgatgt tgccgcacgg tgaagggcgt 420
   attgtgatga cctcatcggt gatgggatta atctccacac cgggtcgtgg cgcttacgcg 480
   gccagtaaat atgcgctgga ggcgtggtca gacgcgctgc gcatggagct gcgccgcagc 540
   gggattaaag tcagcctgat cgaacccggt cccattcgta ctcgcttcac cgacaacgtc 600
   aaccagacgc aaagtgataa accagtcgaa aatcccggca tcgccgcccg ctttacgttg 660
   ggaccggaag cggtggtgga caaagtacgc catgctttta ttagcgagaa gccgaagatg 720
   cgctatccgg taacgctggt gacctgggcg gtaatggtgc ttaagcgcct gctgccgggg 780
   cgcgtgatgg acaaaatatt gcagggg 807

<210> 7

<211>    673
<212>    PRT
<213>    GGPP synthase, crtE

<400>    7
Met Thr Ala Leu Ala Tyr Tyr Gln Ile His Leu Ile Tyr Thr Leu Pro
1               5                   10                  15

Ile Leu Gly Leu Leu Gly Leu Leu Thr Ser Pro Ile Leu Thr Lys Phe
            20                  25                  30

Asp Ile Tyr Lys Ile Ser Ile Leu Val Phe Ile Ala Phe Ser Ala Thr
        35                  40                  45

Thr Pro Trp Asp Ser Trp Ile Ile Arg Asn Gly Ala Trp Thr Tyr Pro
    50                  55                  60

Ser Ala Glu Ser Gly Gln Gly Val Phe Gly Thr Phe Leu Asp Val Pro
65                  70                  75                  80

Tyr Glu Glu Tyr Ala Phe Phe Val Ile Gln Thr Val Ile Thr Gly Leu
                85                  90                  95

Val Tyr Val Leu Ala Thr Arg His Leu Leu Pro Ser Leu Ala Leu Pro
            100                 105                 110

Lys Thr Arg Ser Ser Ala Leu Ser Leu Ala Leu Lys Ala Leu Ile Pro
        115                 120                 125

Leu Pro Ile Ile Tyr Leu Phe Thr Ala His Pro Ser Pro Ser Pro Asp
    130                 135                 140

Pro Leu Val Thr Asp His Tyr Phe Tyr Met Arg Ala Leu Ser Leu Leu
145                 150                 155                 160

Ile Thr Pro Pro Thr Met Leu Leu Ala Ala Leu Ser Gly Glu Tyr Ala
                165                 170                 175

Phe Asp Trp Lys Ser Gly Arg Ala Lys Ser Thr Ile Ala Ala Ile Met
            180                 185                 190

Ile Pro Thr Val Tyr Leu Ile Trp Val Asp Tyr Val Ala Val Gly Gln
        195                 200                 205

Asp Ser Trp Ser Ile Asn Asp Glu Lys Ile Val Gly Trp Arg Leu Gly
    210                 215                 220

Gly Val Leu Pro Ile Glu Glu Ala Met Phe Phe Leu Leu Thr Asn Leu
225                 230                 235                 240

Met Ile Val Leu Gly Leu Ser Ala Cys Asp His Thr Gln Ala Leu Tyr
                245                 250                 255

Leu Leu His Gly Arg Thr Ile Tyr Gly Asn Lys Lys Met Pro Ser Ser
            260                 265                 270

Phe Pro Leu Ile Thr Pro Pro Val Leu Ser Leu Phe Phe Ser Ser Arg
        275                 280                 285

Pro Tyr Ser Ser Gln Pro Lys Arg Asp Leu Glu Leu Ala Val Lys Leu
    290                 295                 300

Leu Glu Glu Lys Ser Arg Ser Phe Phe Val Ala Ser Ala Gly Phe Pro

305                 310                 315                 320

Ser Glu Val Arg Glu Arg Leu Val Gly Leu Tyr Ala Phe Cys Arg Val
                325                 330                 335

Thr Asp Asp Leu Ile Asp Ser Pro Glu Val Ser Ser Asn Pro His Ala
            340                 345                 350

Thr Ile Asp Met Val Ser Asp Phe Leu Thr Leu Leu Phe Gly Pro Pro
        355                 360                 365

Leu His Pro Ser Gln Pro Asp Lys Ile Leu Ser Ser Pro Leu Leu Pro
    370                 375                 380

Pro Ser His Pro Ser Arg Pro Thr Gly Met Tyr Pro Leu Pro Pro Pro
385                 390                 395                 400

Pro Ser Leu Ser Pro Ala Glu Leu Val Gln Phe Leu Thr Glu Arg Val
                405                 410                 415

Pro Val Gln Tyr His Phe Ala Phe Arg Leu Leu Ala Lys Leu Gln Gly
            420                 425                 430

Leu Ile Pro Arg Tyr Pro Leu Asp Glu Leu Leu Arg Gly Tyr Thr Thr
        435                 440                 445

Asp Leu Ile Phe Pro Leu Ser Thr Glu Ala Val Gln Ala Arg Lys Thr
    450                 455                 460

Pro Ile Glu Thr Thr Ala Asp Leu Leu Asp Tyr Gly Leu Cys Val Ala
465                 470                 475                 480

Gly Ser Val Ala Glu Leu Leu Val Tyr Val Ser Trp Ala Ser Ala Pro
                485                 490                 495

Ser Gln Val Pro Ala Thr Ile Glu Glu Arg Glu Ala Val Leu Val Ala
            500                 505                 510

Ser Arg Glu Met Gly Thr Ala Leu Gln Leu Val Asn Ile Ala Arg Asp
        515                 520                 525

Ile Lys Gly Asp Ala Thr Glu Gly Arg Phe Tyr Leu Pro Leu Ser Phe
    530                 535                 540

Phe Gly Leu Arg Asp Glu Ser Lys Leu Ala Ile Pro Thr Asp Trp Thr
545                 550                 555                 560

Glu Pro Arg Pro Gln Asp Phe Asp Lys Leu Leu Ser Leu Ser Pro Ser
                565                 570                 575

Ser Thr Leu Pro Ser Ser Asn Ala Ser Glu Ser Phe Arg Phe Glu Trp
            580                 585                 590

Lys Thr Tyr Ser Leu Pro Leu Val Ala Tyr Ala Glu Asp Leu Ala Lys
        595                 600                 605

His Ser Tyr Lys Gly Ile Asp Arg Leu Pro Thr Glu Val Gln Ala Gly
    610                 615                 620

Met Arg Ala Ala Cys Ala Ser Tyr Leu Leu Ile Gly Arg Glu Ile Lys
625                 630                 635                 640

Val Val Trp Lys Gly Asp Val Gly Glu Arg Arg Thr Val Ala Gly Trp

645                 650                 655

Arg Arg Val Arg Lys Val Leu Ser Val Val Met Ser Gly Trp Glu Gly
            660                 665                 670

Gln

<210>   8
<211>   376
<212>   PRT
<213>   Phytoene synthase, crtYB

<400>   8
Met Asp Tyr Ala Asn Ile Leu Thr Ala Ile Pro Leu Glu Phe Thr Pro
1               5                   10                  15

Gln Asp Asp Ile Val Leu Leu Glu Pro Tyr His Tyr Leu Gly Lys Asn
            20                  25                  30

Pro Gly Lys Glu Ile Arg Ser Gln Leu Ile Glu Ala Phe Asn Tyr Trp
        35                  40                  45

Leu Asp Val Lys Lys Glu Asp Leu Glu Val Ile Gln Asn Val Val Gly
    50                  55                  60

Met Leu His Thr Ala Ser Leu Leu Met Asp Asp Val Glu Asp Ser Ser
65                  70                  75                  80

Val Leu Arg Arg Gly Ser Pro Val Ala His Leu Ile Tyr Gly Ile Pro
                85                  90                  95

Gln Thr Ile Asn Thr Ala Asn Tyr Val Tyr Phe Leu Ala Tyr Gln Glu
            100                 105                 110

Ile Phe Lys Leu Arg Pro Thr Pro Ile Pro Met Pro Val Ile Pro Pro
        115                 120                 125

Ser Ser Ala Ser Leu Gln Ser Ser Val Ser Ser Ala Ser Ser Ser Ser
    130                 135                 140

Ser Ala Ser Ser Glu Asn Gly Gly Thr Ser Thr Pro Asn Ser Gln Ile
145                 150                 155                 160

Pro Phe Ser Lys Asp Thr Tyr Leu Asp Lys Val Ile Thr Asp Glu Met
                165                 170                 175

Leu Ser Leu His Arg Gly Gln Gly Leu Glu Leu Phe Trp Arg Asp Ser
            180                 185                 190

Leu Thr Cys Pro Ser Glu Glu Glu Tyr Val Lys Met Val Leu Gly Lys
        195                 200                 205

Thr Gly Gly Leu Phe Arg Ile Ala Val Arg Leu Met Met Ala Lys Ser
    210                 215                 220

Glu Cys Asp Ile Asp Phe Val Gln Leu Val Asn Leu Ile Ser Ile Tyr
225                 230                 235                 240

Phe Gln Ile Arg Asp Asp Tyr Met Asn Leu Gln Ser Ser Glu Tyr Ala
                245                 250                 255

His Asn Lys Asn Phe Ala Glu Asp Leu Thr Glu Gly Lys Phe Ser Phe
            260                 265                 270

Pro Thr Ile His Ser Ile His Thr Asn Pro Ser Ser Arg Leu Val Ile
        275                 280                 285

Asn Thr Leu Gln Lys Lys Ser Thr Ser Pro Glu Ile Leu His His Cys
    290                 295                 300

Val Asn Tyr Met Arg Thr Glu Thr His Ser Phe Glu Tyr Thr Arg Glu
305                 310                 315                 320

Val Leu Asn Thr Leu Ser Gly Ala Leu Glu Arg Glu Leu Gly Arg Leu
                325                 330                 335

Gln Glu Glu Phe Ala Glu Ala Asn Ser Arg Met Asp Leu Gly Asp Val
            340                 345                 350

Glu Ser Glu Gly Arg Thr Gly Lys Asn Val Lys Leu Glu Ala Ile Leu
        355                 360                 365

Lys Lys Leu Ala Asp Ile Pro Leu
    370                 375

<210> 9
<211> 582
<212> PRT
<213> desaturase, crtI

<400> 9
Met Gly Lys Glu Gln Asp Gln Asp Lys Pro Thr Ala Ile Ile Val Gly
1               5                   10                  15

Cys Gly Ile Gly Gly Ile Ala Thr Ala Ala Arg Leu Ala Lys Glu Gly
            20                  25                  30

Phe Gln Val Thr Val Phe Glu Lys Asn Asp Tyr Ser Gly Gly Arg Cys
        35                  40                  45

Ser Leu Ile Glu Arg Asp Gly Tyr Arg Phe Asp Gln Gly Pro Ser Leu
    50                  55                  60

Leu Leu Leu Pro Asp Leu Phe Lys Gln Thr Phe Glu Asp Leu Gly Glu
65                  70                  75                  80

Lys Met Glu Asp Trp Val Asp Leu Ile Lys Cys Glu Pro Asn Tyr Val
                85                  90                  95

Cys His Phe His Asp Glu Glu Thr Phe Thr Leu Ser Thr Asp Met Ala
            100                 105                 110

Leu Leu Lys Arg Glu Val Glu Arg Phe Glu Gly Lys Asp Gly Phe Asp
        115                 120                 125

Arg Phe Leu Ser Phe Ile Gln Glu Ala His Arg His Tyr Glu Leu Ala
    130                 135                 140

Val Val His Val Leu Gln Lys Asn Phe Pro Gly Phe Ala Ala Phe Leu
145                 150                 155                 160

Arg Leu Gln Phe Ile Gly Gln Ile Leu Ala Leu His Pro Phe Glu Ser
                165                 170                 175

Ile Trp Thr Arg Val Cys Arg Tyr Phe Lys Thr Asp Arg Leu Arg Arg
            180                 185                 190

Val Phe Ser Phe Ala Val Met Tyr Met Gly Gln Ser Pro Tyr Ser Ala
        195                 200                 205

Pro Gly Thr Tyr Ser Leu Leu Gln Tyr Thr Glu Leu Thr Glu Gly Ile
    210                 215                 220

Trp Tyr Pro Arg Gly Gly Phe Trp Gln Val Pro Asn Thr Leu Leu Gln
225                 230                 235                 240

Ile Val Lys Arg Asn Asn Pro Ser Ala Lys Phe Asn Phe Asn Ala Pro
                245                 250                 255

Val Ser Gln Val Leu Leu Ser Pro Ala Lys Asp Arg Ala Thr Gly Val
            260                 265                 270

Arg Leu Glu Ser Gly Glu Glu His His Ala Asp Val Val Ile Val Asn
        275                 280                 285

Ala Asp Leu Val Tyr Ala Ser Glu His Leu Ile Pro Asp Asp Ala Arg
    290                 295                 300

Asn Lys Ile Gly Gln Leu Gly Glu Val Lys Arg Ser Trp Trp Ala Asp
305                 310                 315                 320

Leu Val Gly Gly Lys Lys Leu Lys Gly Ser Cys Ser Ser Leu Ser Phe
                325                 330                 335

Tyr Trp Ser Met Asp Arg Ile Val Asp Gly Leu Gly Gly His Asn Ile
            340                 345                 350

Phe Leu Ala Glu Asp Phe Lys Gly Ser Phe Asp Thr Ile Phe Glu Glu
        355                 360                 365

Leu Gly Leu Pro Ala Asp Pro Ser Phe Tyr Val Asn Val Pro Ser Arg
    370                 375                 380

Ile Asp Pro Ser Ala Ala Pro Glu Gly Lys Asp Ala Ile Val Ile Leu
385                 390                 395                 400

Val Pro Cys Gly His Ile Asp Ala Ser Asn Pro Gln Asp Tyr Asn Lys
                405                 410                 415

Leu Val Ala Arg Ala Arg Lys Phe Val Ile His Thr Leu Ser Ala Lys
            420                 425                 430

Leu Gly Leu Pro Asp Phe Glu Lys Met Ile Val Ala Glu Lys Val His
        435                 440                 445

Asp Ala Pro Ser Trp Glu Lys Glu Phe Asn Leu Lys Asp Gly Ser Ile
    450                 455                 460

Leu Gly Leu Ala His Asn Phe Met Gln Val Leu Gly Phe Arg Pro Ser
465                 470                 475                 480

Thr Arg His Pro Lys Tyr Asp Lys Leu Phe Phe Val Gly Ala Ser Thr
                485                 490                 495

His Pro Gly Thr Gly Val Pro Ile Val Leu Ala Gly Ala Lys Leu Thr
            500                 505                 510

Ala Asn Gln Val Leu Glu Ser Phe Asp Arg Ser Pro Ala Pro Asp Pro
        515                 520                 525

Asn Met Ser Leu Ser Val Pro Tyr Gly Lys Pro Leu Lys Ser Asn Gly
    530                 535                 540

Thr Gly Ile Asp Ser Gln Val Gln Leu Lys Phe Met Asp Leu Glu Arg
545                 550                 555                 560

Trp Val Tyr Leu Leu Val Leu Leu Ile Gly Ala Val Ile Ala Arg Ser
                565                 570                 575

Val Gly Val Leu Ala Phe
            580



<210>    10
<211>    338
<212>    PRT
<213>    Halobacterium salinarum

<400>    10
Met Ala Leu Thr Pro Leu Thr Ala Arg Ala Arg Arg Thr Leu Ala Arg
  1               5                  10                  15

Pro Ala Leu Ala Leu Gly Trp Val Ala Ile Ser Ile Ala Ala Leu Pro
             20                  25                  30

Ala Ile Thr Gly Val Ser Leu Ser Pro Thr Ala Arg Tyr Ala Pro Leu
         35                  40                  45

Val Ala Ser Ala Val Val Phe Gly Met Pro His Gly Ala Ile Asp Tyr
     50                  55                  60

Leu Ala Leu Pro Arg Ala Val Thr Gly Thr Val Thr Val Arg Trp Leu
 65                  70                  75                  80

Ala Val Val Gly Val Leu Tyr Leu Val Leu Gly Gly Gly Tyr Ala Ala
                 85                  90                  95

Ala Trp Phe Phe Ala Pro Val Pro Ala Ala Phe Ala Phe Val Ala Ile
            100                 105                 110

Thr Trp Leu His Trp Gly Gln Gly Asp Leu Tyr Pro Leu Leu Asp Phe
        115                 120                 125

Leu Asp Val Asp Tyr Leu Asp Thr Arg Pro Arg Arg Ala Ala Thr Val
    130                 135                 140

Leu Ile Arg Gly Gly Leu Pro Met Leu Val Pro Leu Leu Gly Phe Pro
145                 150                 155                 160

Glu Arg Tyr Arg Ser Val Val Asp Ala Phe Ala Ala Pro Phe Gly Gly
                165                 170                 175

Ser Val Gly Asp Leu Ala Val Phe Asp Pro Arg Val Arg Leu Trp Leu
            180                 185                 190

Gly Val Ala Phe Ala Ala Ala Thr Val Ala Val Leu Ala Ala Gly Arg
        195                 200                 205

Arg Arg Thr His Ser Pro Gly Ala Trp Arg Asp Ala Ala Glu Thr Leu

210                 215                 220

Leu Leu Trp Val Phe Phe Phe Val Val Pro Pro Val Phe Ala Val Gly
225                 230                 235                 240

Val Tyr Phe Cys Val Trp His Ser Val Arg His Val Ala Arg Ala Ile
                245                 250                 255

Ala Val Asp Gly Ser Val His Pro Ser Leu Arg Ala Gly Asp Ile Leu
            260                 265                 270

Gly Pro Leu Ala Arg Phe Gly Val Glu Ala Ala Pro Met Thr Ala Ala
        275                 280                 285

Ala Leu Ala Leu Gly Gly Val Leu Trp Ala Val Pro Asn Pro Pro Thr
    290                 295                 300

Thr Leu Glu Ser Gly Ala Ala Leu Tyr Leu Val Leu Ile Ala Val Leu
305                 310                 315                 320

Thr Leu Pro His Val Ala Val Val Thr Trp Met Asp Arg Val Gln Gly
                325                 330                 335

Val Leu

<210>    11
<211>    275
<212>    PRT
<213>    Unknown

<220>
<223>    predictet Brp-like protein Blh [uncultured marine bacterium
         66A03]

<400>    11


<210>   12
<211>   269
<212>   PRT
<213>   Escherichia coli

<400>   12
Met Thr His Lys Ala Thr Glu Met Leu Thr Gly Lys Val Met Gln Lys
 1               5                  10                  15

Ser Val Thr Ile Thr Gly Cys Ser Ser Gly Met Gly Leu Glu Ser Ala
            20                  25                  30

Leu Glu Thr Lys Arg Gln Gly Phe His Val Leu Ala Gly Cys Arg Lys
        35                  40                  45

Pro Asp Asp Val Glu Arg Met Asn Ser Met Gly Phe Thr Gly Val Leu
    50                  55                  60

Ile Asp Leu Asp Ser Pro Glu Ser Val Asp Arg Ala Ala Asp Glu Val
65                  70                  75                  80

Ile Ala Thr Thr Asp Asn Cys Leu Tyr Gly Ile Phe Asn Asn Ala Gly
                85                  90                  95

Phe Gly Met Tyr Gly Pro Thr Ser Thr Ile Ser Arg Ala Gln Met Glu
            100                 105                 110

Gln Gln Phe Ser Ala Asn Phe Phe Gly Ala His Gln Leu Thr Met Arg
        115                 120                 125

Thr Leu Pro Ala Met Leu Pro His Gly Glu Gly Arg Ile Val Met Thr
    130                 135                 140

Ser Ser Val Met Gly Leu Ile Ser Thr Pro Gly Arg Gly Ala Tyr Ala
145                 150                 155                 160

Ala Ser Lys Tyr Ala Leu Glu Ala Trp Ser Asp Ala Leu Arg Met Glu
                165                 170                 175

Leu Arg His Ser Gly Ile Lys Val Ser Thr Met Glu Pro Gly Pro Ile
            180                 185                 190

Arg Thr Arg Phe Thr Asp Asn Val Asn Gln Thr Gln Ser Asp Lys Pro
        195                 200                 205

Val Glu Asn Pro Gly Met Ala Ala Arg Phe Thr Leu Gly Pro Glu Ala
    210                 215                 220

Val Val Asp Lys Val Arg His Ala Phe Ile Ser Glu Lys Pro Lys Met
225                 230                 235                 240

Arg Tyr Pro Val Thr Thr Val Thr Trp Ala Val Met Val Thr Lys Arg
                245                 250                 255

Leu Leu Pro Gly Arg Val Met Asp Lys Ile Leu Gln Gly
            260                 265


<210>    13
<211>    70
<212>    DNA
<213>    Artificial Sequence

<220>
<223>    BCMO primer-F


<400>    13
atcgtccttg tatggaagta tcaaagggga cgttcttcac ctccttggaa ccagtcacga        60

cgttgtaaaa                                                               70


<210>    14
<211>    70
<212>    DNA
<213>    Artificial Sequence

<220>
<223>    BCMO primer-R


<400>    14
aatgatttac aatctagtcg caaaaacaag tacagtgctg acgtcccatc aggtttcccg        60

actggaaagc                                                               70


<210>    15
<211>    69
<212>    DNA
<213>    Artificial Sequence

<220>
<223>    ybbO primer-F

<400>    15
aaatccgaac aacagagcat agggtttcgc aaacaaactt aaatatatgc aggtttcccg        60

actggaaag                                                                69



<210>    16
<211>    69
<212>    DNA
<213>    Artificial Sequence

<220>
<223>    ybbO primer-R



<400>    16
gtataatctg tatacataat attataggct ttaccaacaa tggaatttcg ccagtcacga        60

cgttgtaaa                                                                69